(19) Europäisches Patentamt
European Patent Office
Office européen des brevets

(11) Veröffentlichungsnummer: **0 045 016**
**B1**

(12) # EUROPÄISCHE PATENTSCHRIFT

(45) Veröffentlichungstag der Patentschrift: **12.09.84**

(51) Int. Cl.³: **C 07 D 401/12**, A 01 N 43/64

(21) Anmeldenummer: **81105611.8**

(22) Anmeldetag: **17.07.81**

(54) **Substituierte Triazolylalkyl-pyridyl-ether, Verfahren zu ihrer Herstellung sowie ihre Verwendung als Fungizide.**

(30) Priorität: **29.07.80 DE 3028669**

(43) Veröffentlichungstag der Anmeldung: **03.02.82 Patentblatt 82/5**

(45) Bekanntmachung des Hinweises auf die Patenterteilung: **12.09.84 Patentblatt 84/37**

(84) Benannte Vertragsstaaten: **BE CH DE FR GB IT LI NL**

(56) Entgegenhaltungen:
**EP - A - 0 002 678**
**EP - A - 0 032 561**
**DE - A - 2 535 332**
**DE - A - 2 552 967**
**US - A - 4 166 854**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Kraatz, Udo, Dr., Koerner Strasse 6, D-5090 Leverkusen 1 (DE)**
Erfinder: **Stetter, Jörg, Dr., Gellertweg 4, D-5600 Wuppertal 1 (DE)**
Erfinder: **Büchel, Karl Heinz, Prof. Dr., Dabringhauser Strasse 42, D-5093 Burscheid (DE)**
Erfinder: **Frohberger, Paul-Ernst, Dr., Willi-Baumeister-Strasse 5, D-5090 Leverkusen 1 (DE)**
Erfinder: **Brandes, Wilhelm, Dr., Eichendorffstrasse 3, D-5653 Leichlingen 1 (DE)**

**EP 0 045 016 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue substituierte Triazolylalkyl-pyridyl-ether,mehrere Verfahren zu ihrer Herstellung sowie ihre Verwendung als Fungizide.

Es ist bereits bekannt geworden, dass bestimmte 3,3-Dimethyl-1-pyridyloxy-1-triazolylbutan-2-one eine gute fungizide Wirksamkeit besitzen (vgl. DE-OS 2 756 269). Deren Wirkung ist jedoch, insbesondere bei niedrigen Aufwandmengen und -konzentrationen, nicht immer ganz befriedigend.

Es wurden nun neue substituierte Triazolylalkyl-pyridyl-ether der Formel (I)

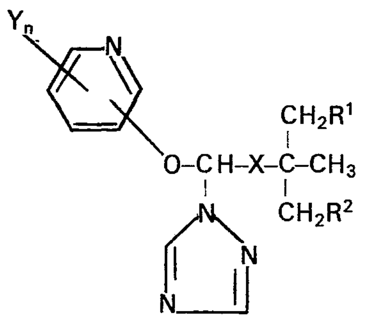

(I)

in welcher

$R^1$ für Halogen steht,

$R^2$ für Wasserstoff oder Halogen steht,

X für die Ketogruppe oder eine CH(OH)-Gruppierung steht,

Y für Halogen, für Alkyl oder Alkoxy mit 1 bis 4 Kohlenstoffatomen oder für Cyano steht und

n für die Zahlen 0, 1, 2, 3 oder 4 steht, und deren physiologisch verträglichen Säureadditions-Salze sowie Metallsalz-Komplexe gefunden.

Diejenigen Verbindungen der Formel (I), in welchen X für die CH(OH)-Gruppe steht, besitzen zwei asymmetrische Kohlenstoffatome; sie können deshalb in den beiden geometrischen Isomeren (erythro- und threo-Form) vorliegen, die in unterschiedlichen Mengenverhältnissen anfallen können. In beiden Fällen liegen sie als optische Isomeren vor. Sämtliche Isomeren werden erfindungsgemäss beansprucht.

Weiterhin wurde gefunden, dass man die substituierten Triazolylalkyl-pyridyl-ether der Formel (I) erhält, wenn man

a) Triazolylhalogenketone der Formel (II)

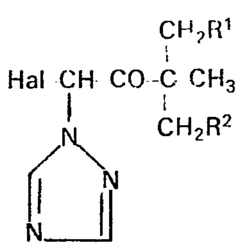

(II)

in welcher

$R^1$ und $R^2$ die oben angegebene Bedeutung haben und

Hal für Chlor oder Brom steht, mit Pyridinolen der Formel (III)

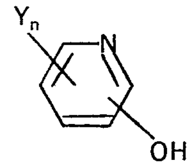

(III)

in welcher

Y und n die oben angegebene Bedeutung haben, in Gegenwart eines Säurebindemittels und in Gegenwart eines Verdünnungsmittels umsetzt, oder

b) Halogenetherketone der Formel (IV)

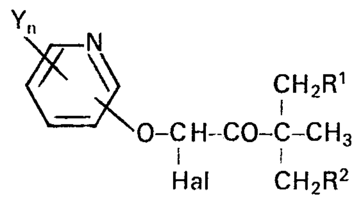

(IV)

in welcher

Hal, $R^1$, $R^2$, Y und n die oben angegebene Bedeutung haben, mit 1,2,4-Triazol in Gegenwart eines Säurebindemittels und gegebenenfalls in Gegenwart eines Verdünnungsmittels umsetzt; und

c) gegebenenfalls die nach den Verfahrensvarianten (a) und (b) erhaltenen Keto-Derivate der Formel (Ia)

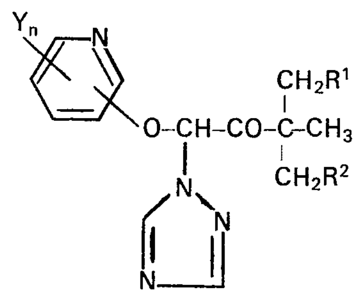

(Ia)

in welcher

$R^1$, $R^2$, Y und n die oben angegebene Bedeutung haben, nach bekannten Methoden in üblicher Weise reduziert.

An die so erhaltenen Verbindungen der Formel (I) kann gegebenenfalls anschliessend eine Säure oder ein Metallsalz addiert werden.

Die neuen substituierten Triazolyl-pyridyl-ether der Formel (I) weisen starke fungizide Eigenschaften auf. Dabei zeigen überraschenderweise die erfindungsgemässen Verbindungen eine bessere fungizide Wirksamkeit als die aus dem Stand der Technik bekannten 3,3-Dimethyl-1-pyridyloxy-1-triazolyl-butan-2-one, welche chemisch und wirkungsmässig die naheliegendsten Verbindungen sind. Die erfindungsgemässen Stoffe stellen somit eine Bereicherung der Technik dar.

Die erfindungsgemässen substituierten Triazolyl-pyridyl-ether sind durch die Formel (I) allgemein definiert. In dieser Formel steht $R^1$ vorzugsweise für Fluor oder Chlor und $R^2$ vorzugsweise für Wasserstoff oder $R^1$ und $R^2$ sind gleich und stehen vorzugsweise für Fluor oder Chlor.

Sofern n Zahlenwerte von 2 bis 4 annimmt,

kann der Rest Y gleiche oder verschiedene Bedeutungen annehmen.

Verwendet man beispielsweise 1-Brom-3,3-dimethyl-4-fluor-1-(1,2,4-triazol-1-yl)-butan-2-on und 6-Chlor-pyridin-2-ol als Ausgangsstoffe, so kann der Reaktionsablauf durch das folgende Formelschema wiedergegeben werden (Verfahren a):

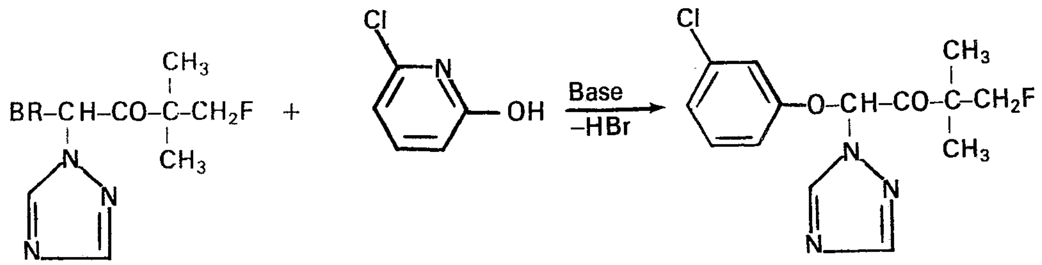


Verwendet man beispielsweise 1-Brom-1-(6-chlor-pyridin-2-yl-oxy)-3,3-dimethyl-4-fluorbutan-2-on und 1,2,4-Triazol als Ausgangsstoffe, so kann der Reaktionsablauf durch das folgende Formelschema wiedergegeben werden (Verfahren b):

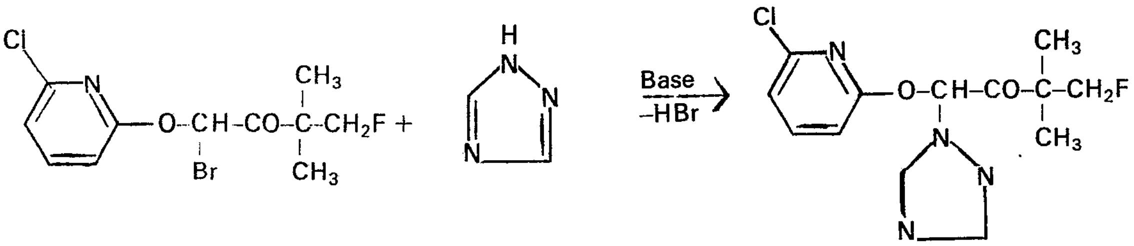


Verwendet man beispielsweise 1-(6-Chlor-pyridin-2-yl-oxy)-3,3-dimethyl-4-fluor-1-(1,2,4-triazol-1-yl)-butan-2-on und Natriumborhydrid als Ausgangsstoffe, so kann der Reaktionsablauf durch das folgende Formelschema wiedergegeben werden (Verfahren c):

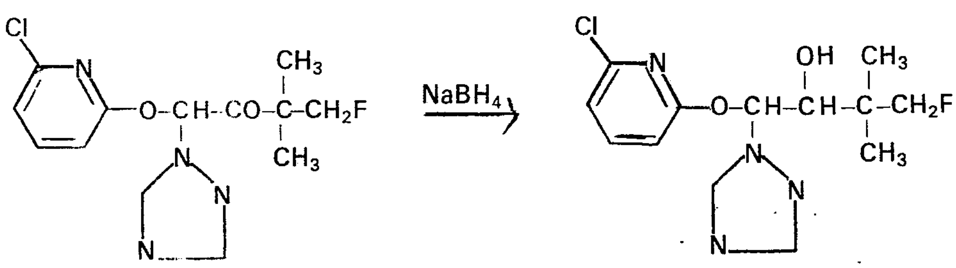


Die als Ausgangsstoffe für die Verfahrensvariante (a) zu verwendenden Triazolylhalogenketone sind durch die Formel (II) allgemein definiert. In dieser Formel stehen $R^1$ und $R^2$ vorzugsweise für die Reste, die bereits im Zusammenhang mit der Beschreibung der erfindungsgemässen Stoffe der Formel (I) vorzugsweise für diese Substituenten genannt wurden.

Die Triazolylhalogenketone der Formel (II) sind noch nicht bekannt; sie können jedoch in allgemein bekannter Art und Weise erhalten werden, indem man Halogenide der Formel (V)

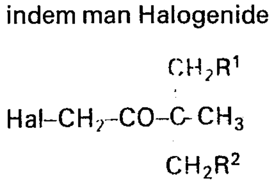
(V)

in welcher

$R^1$, $R^2$ und Hal die oben angegebene Bedeutung haben, mit 1,2,4-Triazol in Gegenwart eines Säurebindemittels, wie z.B. Kaliumcarbonat, und in Gegenwart eines inerten organische Lösungsmittels, wie z.B. Aceton, bei Temperaturen zwischen 60 und 120 °C umsetzt. Eines der beiden aktiven Wasserstoffatome wird anschliessend in üblicher Weise gegen Chlor oder Brom ausgetauscht. Die Triazolylhalogenketone der Formel (II) können direkt weiter umgesetzt werden.

Die Halogenide der Formel (V) sind bekannt (vgl. DE-OS 2 632 603 und DE-OS 2 843 767).

Als Ausgangsstoffe der Formel (II) seien genannt:
1-Brom(chlor)-3,3-dimethyl-4-fluor-1-(1,2,4-triazol-1-yl)-butan-2-on,
1-Brom(chlor)-4-chlor-3,3-dimethyl-1-(1,2,4-triazol-1-yl)-butan-2-on,
3,3-Bisfluormethyl-1-brom(chlor)-1-(1,2,4-triazol-1-yl)-butan-2-on,
3,3-Bischlormethyl-1-brom(chlor)-1-(1,2,4-triazol-1-yl)-butan-2-on.

Die ausserdem für die Verfahrensvariante (a)

als Ausgangsstoffe zu verwendenden Pyridinole sind durch die Formel (III) allgemein definiert. Gegebenenfalls werden die Pyridinole der Formel (III) auch in Form ihrer Silbersalze eingesetzt.

Die Pyridinole der Formel (III) sind allgemein bekannte Verbindungen der organischen Chemie.

Als Beispiele seien genannt:
2-Hydroxy-pyridin, 3-Hydroxy-pyridin, 4-Hydroxy-pyridin, 2-Hydroxy-6-chlor-pyridin, 3-Hydroxy-5-chlor-pyridin, 2-Hydroxy-4-chlor-pyridin, 2-Hydroxy-3-chlor-pyridin, 2-Hydroxy-6-brom-pyridin, 2-Hydroxy-5-brom-pyridin, 2-Hydroxy-4-brom-pyridin, 2-Hydroxy-3-brom-pyridin, 2-Hydroxy-6-methyl-pyridin, 2-Hydroxy-5-methyl-pyridin, 2-Hydroxy-4-methyl-pyridin, 2-Hydroxy-3-methyl-pyridin, 2-Hydroxy-6-fluor-pyridin, 2-Hydroxy-5-fluor-pyridin, 2-Hydroxy-4-fluor-pyridin, 2-Hydroxy-3-fluor-pyridin, 3-Hydroxy-2-chlor-pyridin, 3-Hydroxy-2-brom-pyridin, 3-Hydroxy-2-fluor-pyridin, 3-Hydroxy-2-jod-pyridin, 3-Hydroxy-2-methoxy-pyridin, 3-Hydroxy-6-chlor-pyridin, 3-Hydroxy-5-chlor-pyridin, 4-Hydroxy-2-chlor-pyridin, 4-Hydroxy-pyridin, 4-Hydroxy-3-chlor-pyridin, 2-Hydroxy-3,5,6-trichlorpyridin, 2-Hydroxy-3-cyano-5,6-dichlor-4-methyl-pyridin, 2-Hydroxy-5-brom-6-chlor-pyridin, 2-Hydroxy-5-chlor-4,6-dimethyl-3-cyano-pyridin, 2-Hydroxy-3,5-dichlor-pyridin, 2-Hydroxy-5-brom-4,6-dimethyl-3-cyano-pyridin, 3-Hydroxy-2,6-dijodpyridin, 2-Hydroxy-3,5-dibrom-6-chlor-pyridin, 2-Hydroxy-5-brom-6-chlor-3-cyano-4-methylpyridin.

Die für die Verfahrensvariante (b) als Ausgangsstoffe zu verwendenden Halogentherketone sind durch die Formel (IV) allgemein definiert. In dieser Formel stehen $R^1$ und $R^2$ vorzugsweise für die Reste, die bereits im Zusammenhang mit der Beschreibung der erfindungsgemässen Stoffe der Formel (I) vorzugsweise für diese Substituenten genannt wurden.

Die Halogenetherketone der Formel (IV) sind noch nicht bekannt; können aber nach bekannten Verfahren hergestellt werden, indem man Pyridinole der Formel (III) mit Halogeniden der Formel (V) in Gegenwart eines Säurebindemittels, wie z.B. Kaliumcarbonat, und in Gegenwart eines inerten organischen Lösungsmittels, wie z.B. Aceton, bei Temperaturen zwischen 60 und 120 °C umsetzt. Einer der beiden aktiven Wasserstoffatome wird anschliessend in üblicher Weise gegen Chlor oder Brom ausgetauscht.

Für die erfindungsgemässen Umsetzungen gemäss Verfahrensvarianten (a) und (b) kommen als Verdünnungsmittel inerte organische Lösungsmittel infrage. Hierzu gehören vorzugsweise Ketone, wie Diethylketon, insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; Benzol; Formamide; wie insbesondere Dimethylformamid; und halogenierte Kohlenwasserstoffe.

Die Umsetzungen nach Verfahren (a) und (b) werden in Gegenwart eines Säurebinders vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat oder wie Silbercarbonat, oder wie niedere tertiäre Alkylamine, Cycloalkylamine oder Aralkylamine, beispielsweise Triethylamin, Dimethylbenzylamin; oder wie Pyridin und Diazabicyclooctan.

Bei der Verfahrensvariante (b) kann auch ein entsprechender Überschuss an Azol verwendet werden.

Die Reaktionstemperaturen können bei den Verfahren (a) und (b) in einem grösseren Bereich variiert werden. Im allgemeinen arbeitet man zwischen etwa 20 bis etwa 150 °C, vorzugsweise bei 60 bis 120 °C. Bei Anwesenheit eines Lösungsmittels wird zweckmässigerweise beim Siedepunkt des jeweiligen Lösungsmittels gearbeitet.

Bei der Durchführung des erfindungsgemässen Verfahrens (a) bzw. (b) setzt man auf 1 Mol der Verbindungen der Formel (II) bzw. (IV) vorzugsweise 1 bis 2 Mol Pyridinol der Formel (III) bzw. 1 bis 2 Mol Azol und jeweils 1 bis 2 Mol Säurebinder ein. Zur Isolierung der Verbindungen der Formel (I) wird das Lösungsmittel abdestilliert und der Rückstand entweder mit Wasser versetzt und kräftig gerührt, wobei das Reaktionsprodukt durchkristallisiert, oder mit einem Gemisch aus einem organischen Solvens und Wasser aufgenommen, die organische Phase abgetrennt, mit Wasser gewaschen, über Natriumsulfat getrocknet und im Vakuum vom Lösungsmittel befreit. Der Rückstand wird gegebenenfalls durch Destillation oder Umkristallisation gereinigt.

Die erfindungsgemässe Reduktion gemäss Verfahrensvariante (c) erfolgt in üblicher Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemässe Umsetzung polare organische Lösungsmittel infrage. Hierzu gehören vorzugsweise Alkohole, wie Methanol, Ethanol, Butanol, Isopropanol, und Ether, wie Diethylether oder Tetrahydrofuran.

Die Reaktion wird im allgemeinen bei 0 bis 30 °C, vorzugsweise bei 0 bis 20 °C durchgeführt. Hierzu setzt man auf 1 Mol des Ketons der Formel (Ia) etwa 1 Mol eines komplexen Hydrids, wie Natriumhydrid oder Lithiumalanat, ein. Zur Isolierung der reduzierten Verbindungen der Formel (I) wird der Rückstand in verdünnter Salzsäure aufgenommen, anschliessend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemässe Umsetzung bevorzugt Alkohole, wie Isopropanol, oder inerte Kohlenwasserstoffe, wie Benzol, infrage. Die Reaktionstemperaturen

können wiederum in einem grösseren Bereich variiert werden; im allgemeinen arbeitet man zwischen 20 und 120 °C, vorzugsweise bei 50 bis 100 °C. Zur Durchführung der Reaktion setzt man auf 1 Mol des Ketons der Formel (Ia) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die entstandene Aluminium-Verbindung mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure, Milchsäure, sowie Sulfonsäuren, wie z.B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisation reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung von Getreidekrankheiten, wie Getreidemehltau und Gerstenmehltau, von Venturia-Arten, wie gegen den Erreger des Apfelschorfs (Fusicladium dendriticum), und von Reiskrankheiten, wie gegen Pellicularia sasakii, eingesetzt werden. Besonders hervorzuheben ist, dass die erfindungsgemässen Wirkstoffe nicht nur eine protektive Wirkung haben, sondern teilweise auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanzen zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Parafinne, z.B. Erdölfraktionen, Alkohole, wie Butanol oder Glykol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder

Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Herstellungsbeispiele

### Beispiel 1

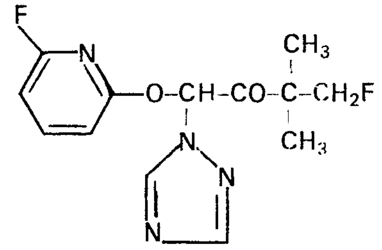

(Verfahren a)

18,5 g (0,1 Mol) 3,3-Dimethyl-4-fluor-1-(1,2,4-triazol-1-yl)-butan-2-on werden in 150 ml Eisessig gelöst und nach Zusatz von 8,2 g (0,1 Mol) Natriumacetat bei 45 °C tropfenweise mit 16 g (0,1 Mol) Brom bis zur völligen Entfärbung versetzt. Anschliessend gibt man in Eiswasser und extrahiert mit Chloroform. Die vereinigten Chloroformextrakte werden mit Natriumhydrogencarbonatlösung gewaschen, über Magnesiumsulfat getrocknet und eingeengt. Das zurückbleibende ölige 1-Brom-3,3-dimethyl-4-fluor-1-(1,2,4-triazol-1-yl)-butan-2-on wird in 50 ml Acetonitril gelöst und unter Rühren mit 11,3 g (0,1 Mol) 6-Fluor-2-hydroxy-pyridin sowie 10,5 g (0,1 Mol) Triethylamin, beides gelöst in 120 ml Acetonitril, versetzt. Man lässt 1 Stunde unter Rückfluss rühren. Danach wird durch Abdestillieren des Lösungsmittels im Vakuum eingeengt und der Rückstand mit Wasser verrührt. Der entstehende kristalline Niederschlag wird abgesaugt und aus Cyclohexan umkristallisiert. Man erhält 20 g (68% der Theorie) 3,3-Dimethyl-4-fluor-1-(6-fluor-pyridin-2-yl-oxy)-1-(1,2,4-triazol-1-yl)-butan-2-on vom Schmelzpunkt 116 °C.

### Herstellung des Vorproduktes

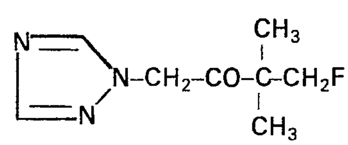

In die siedende Lösung von 77 g (1,1 Mol) 1,2,4-Triazol und 210 g (1,5 Mol) Kaliumcarbonat in 500 ml Aceton tropft man langsam 197 g (1 Mol) 1-Brom-3,3-dimethyl-4-fluor-2-butanon so zu, dass nach Entfernen des Heizbades die exotherme Reaktion die Lösung schwach am Sieden hält. Anschliessend hält man noch 3 Stunden unter Rückfluss, saugt die anorganischen Salze ab und engt das Filtrat im Vakuum ein. Der Rückstand wird mit Chloroform/Wasser aufgenommen, die organische Phase abgetrennt und eingedampft. Der verbliebene Rückstand wird im Vakuum destilliert und das Destillat mit Cyclohexan zur Kristallisation gebracht. Man erhält 112 g (55% der Theorie) 3,3-Dimethyl-4-fluor-1-(1,2,4-triazol-1-yl)-butan-2-on vom Schmelzpunkt 59 °C.

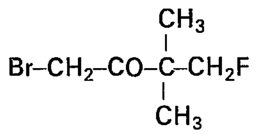

In eine Mischung aus 354 g (3 Mol) 3,3-Dimethyl-4-fluor-2-butanon (siehe Beispiel 1) und 2000 ml Ether werden bei 20 bis 30 °C unter Kühlen und Rühren 480 g Brom langsam eingetropft. Die gelbliche Lösung wird noch 1 Stunde bei 20 °C nachgerührt und anschliessend vorsichtig mit 500 ml Wasser versetzt. Die Etherphase wird abgetrennt, mehrmals mit Wasser gewaschen und über Natriumsulfat getrocknet. Nach Abdestillieren des Lösungsmittels wird der Rückstand im Wasserstrahlvakuum destilliert. Man erhält 472 g (80% der Theorie) 1-Brom-3,3-dimethyl-4-fluor-2-butanon vom Siedepunkt 80 bis 90 °C/11 mm Hg-Säule.

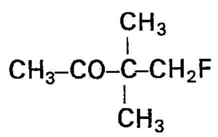

Zu der in einem Dreihals-Rührkolben mit absteigendem Kühler befindlichen Suspension von 23,2 g (0,4 Mol) trockenem Kaliumfluorid in 400 ml dest. Tetraethylenglykol werden bei 160 °C und 20 mbar 38,8 g (0,2 Mol) 2,2-Dimethyl-3-oxobutyl-methansulfonat im Verlauf von 2-Stunden zugetropft und 2 weitere Stunden nachgerührt. An einem absteigenden Kondensator und in einer nachgeschalteten Tiefkühlfalle wird das herausdestillierte Reaktionsprodukt kondensiert und gesammelt. Man gewinnt so 20,9 g (0,177 Mol, das sind 89% der Theorie) 3,3-Dimethyl-4-fluor-2-butanon, das unter Normaldruck bei 130 bis 134 °C siedet.

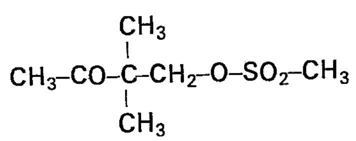

232 g (2 Mol) 3,3-Dimethyl-4-hydroxy-2-butanon (z. Herstellung vgl. Beilstein H1 E III 3239, IV 4030 und Bull. Soc. Chim. France 1964, 2849) werden in 700 ml abs. Pyridin bei 0 bis 5 °C mit 229 g (2 Mol) Methansulfochlorid umgesetzt. Nach 12 Stunden Stehen bei 20 °C wird mit Methylenchlorid verdünnt und mit Eiswasser ausgeschüttelt. Die organische Phase wird getrocknet, im Vakuum vom Lösungsmittel befreit und über eine Kolonne fraktioniert. Bei $Kp_{0,12}$ 106 bis 120 °C isoliert man 332 g (das sind 86% der Theorie) 2,2-Dimethyl-3-oxo-butyl-methansulfonat.

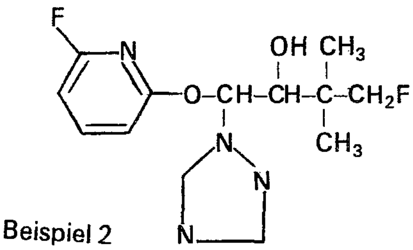

Beispiel 2

(Verfahren c)

10 g (34 Mol) 3,3-Dimethyl-4-fluor-1-(6-fluor-pyridin-2-yl-oxy)-1-(1,2,4-triazol-1-yl)-butan-2-on (Beispiel 1) werden in 100 ml Methanol gelöst und mit 0,7 g (17 Mol) Natriumborhydrid versetzt. Man lässt 10 Minuten unter Rückfluss rühren, engt ein und verteilt den Rückstand zwischen Methylenchlorid/Wasser. Die organische Phase wird abgetrennt, mit Wasser gewaschen, über Natriumsulfat getrocknet und eingeengt. Man erhält 9,3 g (90% der Theorie) wachsartiges 3,3-Dimethyl-4-fluor-1-(6-fluor-pyridin-2-yl-oxy)-1-(1,2,4-triazol-1-yl)-butan-2-ol.

Analog und gemäss den Verfahren (a), (b) und (c) werden die folgenden Verbindungen der Formel (I)

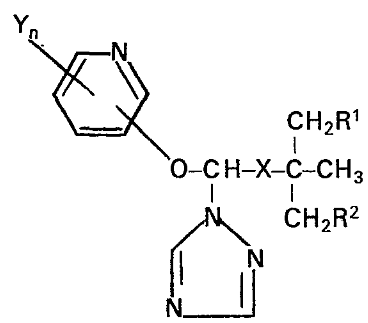 (I)

| Beispiel Nr. | 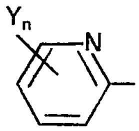 | X | $R^1$ | $R^2$ | Schmelzpunkt ( °C) |
|---|---|---|---|---|---|
| 3 | 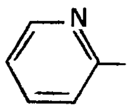 | CO | F | H | 62 |
| 4 | 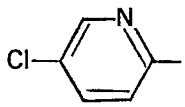 | CO | F | H | 108–110 |
| 5 | 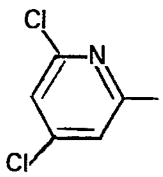 | CO | F | H | 102–104 |
| 6 | 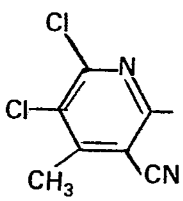 | CO | F | H | 120 |
| 7 | 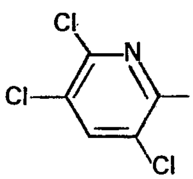 | CO | F | H | 136 |
| 8 | 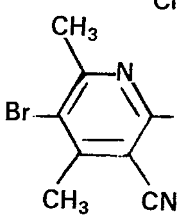 | CO | F | H | 153 |
| 9 | 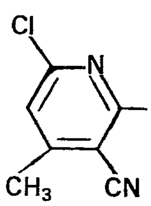 | CO | F | H | 105 |
| 10 | 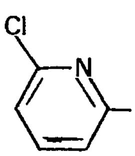 | CO | Cl | H | 63–65 |
| 11 | 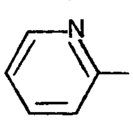 | CO | Cl | H | 58 |

(Fortsetzung)

| Beispiel Nr. | 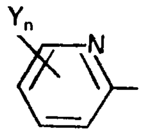 | X | $R^1$ | $R^2$ | Schmelzpunkt ( °C) |
|---|---|---|---|---|---|
| 12 | 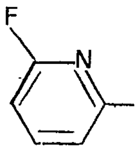 | CO | Cl | H | 89 |
| 13 | 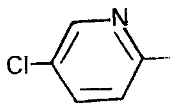 | CO | Cl | H | 100 |
| 14 | 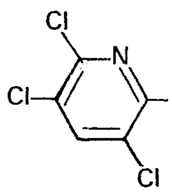 | CO | Cl | H | 126 |
| 15 | 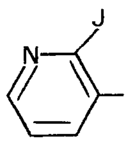 | CO | F | H | 100 |
| 16 | 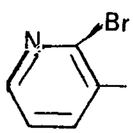 | CO | F | H | 88 |
| 17 | 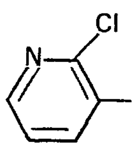 | CO | F | H | Öl |
| 18 | 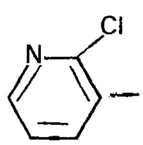 | CO | Cl | H | Öl |
| 19 | 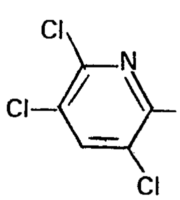 | CH(OH) | F | H | 78 |
| 20 | 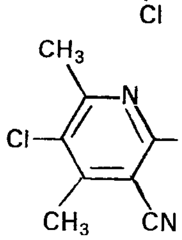 | CH(OH) | F | H | 171 |

(Fortsetzung)

| Beispiel Nr. | 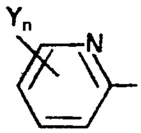 | X | $R^1$ | $R^2$ | Schmelzpunkt ( °C) |
|---|---|---|---|---|---|
| 21 | 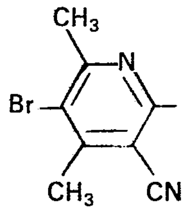 | CH(OH) | F | H | 176 |
| 22 | 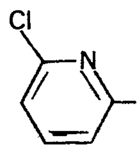 | CH(OH) | Cl | H | Öl |
| 23 | 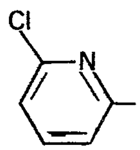 | CH(OH) | F | H | Öl |
| 24 | 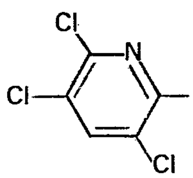 | CH(OH) | Cl | H | 135–137 |
| 25 | 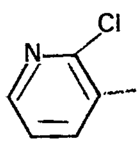 | CH(OH) | F | H | 110–120 |
| 26 | 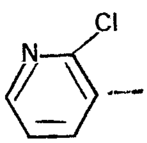 | CH(OH) | Cl | H | 108–118 |
| 27 | 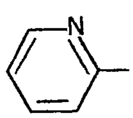 | CO | F | F | 54 |
| 28 | 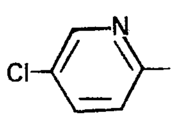 | CO | F | F | 101–106 |
| 29 | 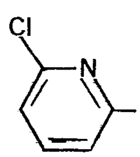 | CO | F | F | 76 |

(Fortsetzung)

| Beispiel Nr. | 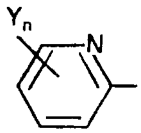 | X | $R^1$ | $R^2$ | Schmelzpunkt (°C) |
|---|---|---|---|---|---|
| 30 | 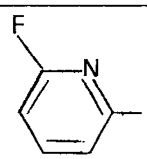 | CO | F | F | 100 |
| 31 | 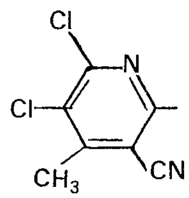 | CO | F | F | 126 |
| 32 | 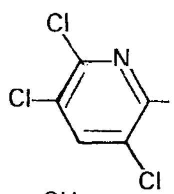 | CO | F | F | 136 |
| 33 | 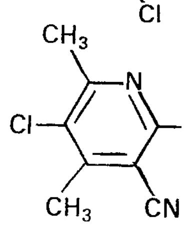 | CO | F | F | 110 |
| 34 | 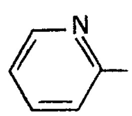 | CO | Cl | Cl | Öl |
| 35 | 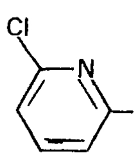 | CO | Cl | Cl | 65 |
| 36 | 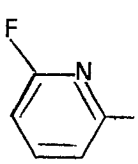 | CO | Cl | Cl | 76 |
| 37 | 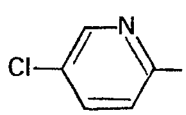 | CO | Cl | Cl | 98 |
| 38 | 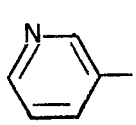 | CO | F | F | Öl |

(Fortsetzung)

| Beispiel Nr. | 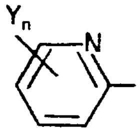 | X | $R^1$ | $R^2$ | Schmelzpunkt (°C) |
|---|---|---|---|---|---|
| 39 | 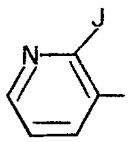 | CO | F | F | 82 |
| 40 | 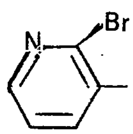 | CO | F | F | 45 |
| 41 | 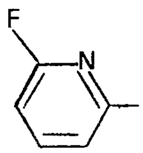 | CH(OH) | F | F | 102–106 |
| 42 | 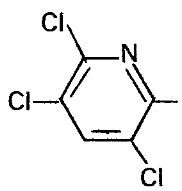 | CH(OH) | F | F | 140–142 |
| 43 | 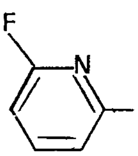 | CH(OH) | Cl | Cl | Harz |
| 44 | 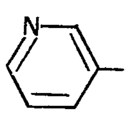 | CH(OH) | Cl | Cl | Harz |

Anwendungsbeispiele

In den nachfolgenden Beispielen werden die nachstehend angegebenen Verbindungen als Vergleichssubstanzen eingesetzt:

(A) 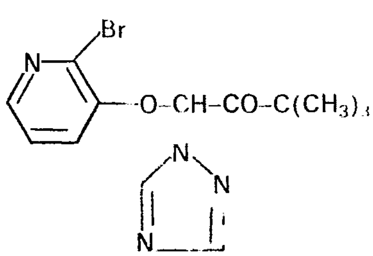

(B) 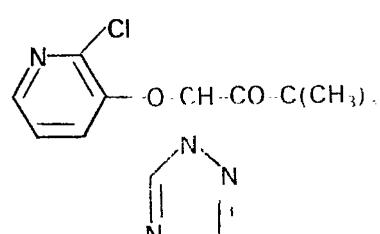

(C) 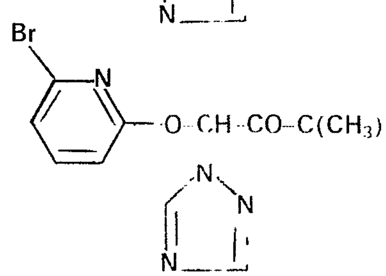

Beispiel A

Erysiphe-Test (Gerste)/protektiv

Lösungsmittel: 100 Gewichtsteile Dimethylformamid

Emulgator: 0,25 Gewichtsteile Alkyl-arylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20 °C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 1, 3, 4, 15, 16, 5, 10, 27, 28, 29, 30, 31, 32, 38, 39, 40, 2, 23, 25, 20, 22 und 26.

Beispiel B

Gerstenmehltau-Test (Erysiphe graminis var. hordei)/systemisch (pilzliche Getreidesprosskrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 × 12 Korn in Blumentöpfe 2 cm tief in ein Gemisch aus einen Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21–22 °C und 80–90% rel. Luftfeuchte und 16-stündiger Belichtung weiter kultiviert. Innerhalb von 6 Tagen bilden sich an den Blättern die typischen Mehltaupusteln aus.

Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Eine deutliche Überlegenheit in der Wirksamkeit gegenüber dem Stand der Technik zeigen bei diesem Test z.B. die Verbindungen gemäss folgender Herstellungsbeispiele: 1, 5, 17, 10 und 23.

Beispiel C

Fusicladium-Test (Apfel)/protektiv

| | |
|---|---|
| Lösungsmittel: | 4,7 Gewichtsteile Aceton |
| Emulgator: | 0,3 Gewichtsteile Alkylarylpolyglykolether |
| Wasser: | 95,0 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20 °C und einer relativen Luftfeuchtigkeit von 70% im Gewächshaus. Anschliessend werden sie mit einer wässrigen Konidiensuspension des Apfelschorferregers (Fusicladium dendriticum) inokuliert und 18 Stunden lang in einer Feuchtkammer bei 18 bis 20 °C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, dass die Pflanzen vollständig befallen sind.

Eine deutliche Überlegenheit in der Wirksamkeit gegenüber dem Stand der Technik zeigen bei diesem Test z.B. die Verbindungen gemäss folgender Herstellungsbeispiele: 1, 22, 23 und 17.

**Patentansprüche**

1. Substituierte Triazolylalkyl-pyridyl-ether der Formel (I)

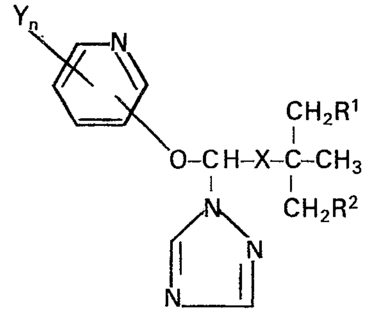

(I)

in welcher

$R^1$ für Halogen steht,

$R^2$ für Wasserstoff oder Halogen steht,

X für die Ketogruppe oder eine CH(OH)-Gruppierung steht,

Y für Halogen, für Alkyl oder Alkoxy mit 1 bis 4 Kohlenstoffatomen oder für Cyano steht und

n für die Zahlen 0, 1, 2, 3 oder 4 steht, und deren physiologisch verträglichen Säureadditions-Salze sowie Metallsalz-Komplexe.

2. Substituierte Triazolylalkyl-pyridyl-ether der Formel (I) in Anspruch 1, wobei

$R^1$ für Fluor oder Chlor steht,

$R^2$ für Wasserstoff steht oder dieselbe Bedeutung wie $R^1$ hat,

X für die Ketogruppe oder eine CH(OH)-Gruppierung steht,

Y für Halogen, geradkettiges oder verzweigtes Alkyl und/oder Alkoxy mit jeweils 1 bis 4 Kohlenstoffatomen oder für Cyano steht, und

n für die Zahlen 0, 1, 2, 3 oder 4 steht, und deren physiologisch verträglichen Säureadditions-Salze sowie Metallsalz-Komplexe.

3. Verfahren zur Herstellung von substituierten Triazolylalkyl-pyridyl-ethern der Formel (I)

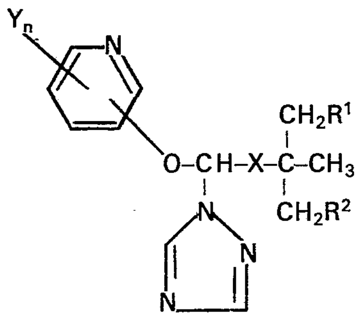

(I)

in welcher

$R^1$ für Halogen steht,

$R^2$ für Wasserstoff oder Halogen steht,

X für die Ketogruppe oder eine CH(OH)-Gruppierung steht,

Y für Halogen, Alkyl, Alkoxy oder Cyano steht und

n für die Zahlen 0,1, 2, 3 oder 4 steht, und deren physiologisch verträglichen Säureadditions-Salzen sowie Metallsalz-Komplexen, dadurch gekennzeichnet, dass man

a) Triazolylhalogenketone der Formel (II)

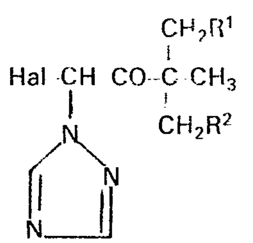

(II)

in welcher

$R^1$ und $R^2$ die oben angegebene Bedeutung haben und

Hal für Chlor oder Brom steht, mit Pyridinolen der Formel (III)

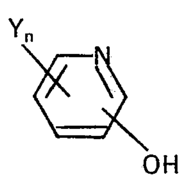

(III)

in welcher

Y und n die oben angegebene Bedeutung haben, in Gegenwart eines Säurebindemittels und in Gegenwart eines Verdünnungsmittels umsetzt, oder

b) Halogenetherketone der Formel (IV)

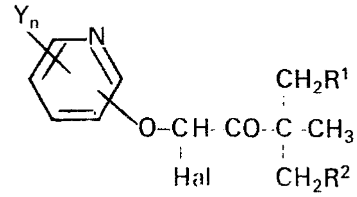

(IV)

in welcher

Hal, $R^1$, $R^2$, Y und n die oben angegebene Bedeutung haben, mit 1,2,4-Triazol in Gegenwart eines Säurebindemittels und gegebenenfalls in Gegenwart eines Verdünnungsmittels umsetzt; und

c) gegebenenfalls die nach den Verfahrensvarianten (a) und (b) erhaltenen Keto-Derivate der Formel (Ia)

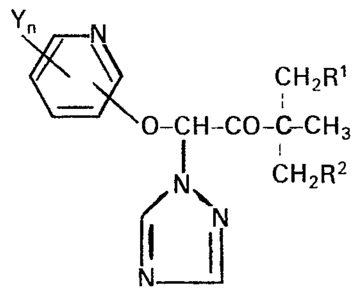

(Ia)

in welcher

$R^1$, $R^2$, Y und n die oben angegebene Bedeutung haben, nach bekannten Methoden in üblicher Weise reduziert, und an die so erhaltenen Verbindungen der Formel (I) noch gegebenenfalls anschliessend eine Säure oder ein Metallsalz addiert.

4. Fungizide Mittel, gekennzeichnet durch einen Gehalt an mindestens einem substituierten Triazolylalkyl-pyridyl-ether der Formel (I).

5. Verwendung von substituierten Triazolylalkyl-pyridyl-ethern der Formel (I) zur Bekämpfung von phytopathogenen Pilzen.

**Revendications**

1. Triazolylalcoyl-pyridyl-éthers substitués de formule (I)

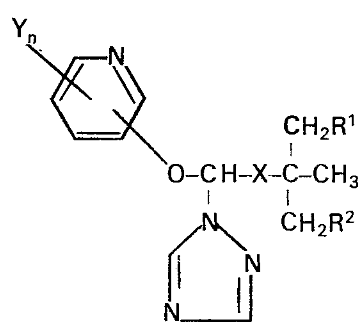

(I)

dans laquelle

$R^1$ représente de l'halogène,

$R^2$ de l'hydrogène ou de l'halogène,

X le groupe cétonique ou un groupement CH(OH),

Y de l'halogène, un alcoyle ou alcoxy ayant 1 à 4 atomes de carbone ou un cyano et

n les nombres 0, 1, 2, 3 ou 4, et leurs sels d'addition d'acides et complexes avec des sels métalliques physiologiquement compatibles.

2. Triazolylalcoyl-pyridyl-éthers substitués de formule (I) selon la revendication 1, dans laquelle

$R^1$ représente du fluor ou du chlore,

$R^2$ de l'hydrogène ou a la même signification que $R^1$,

X le groupe cétonique ou un groupement CH(OH),

Y de l'halogène, un alcoyle et/ou alcoxy à chaîne droite ou ramifiée ayant chacun 1 à 4 atomes de carbone ou un cyano, et

n les nombres 0, 1, 2, 3 ou 4, et leurs sels d'addition d'acides et complexes avec des sels métalliques physiologiquement compatibles.

3. Procédé de préparation de triazolylalcoyl-pyridyl-éthers substitués de formule (I)

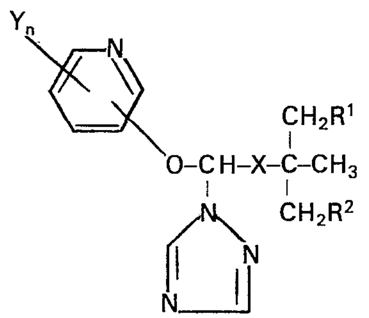

(I)

dans laquelle

$R^1$ représente de l'halogène,

$R^2$ de l'hydrogène ou de l'halogène,

X le groupe cétonique ou un groupement CH(OH),

Y de l'halogène, un alcoyle, alcoxy ou cyano et

n les nombres 0, 1, 2, 3 ou 4, et de leurs sels d'addition d'acides et complexes avec des sels métalliques physiologiquement compatibles, caractérisé en ce que

a) on fait réagir des triazolylhalogénocétones de formule (II)

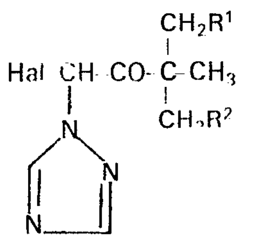

(II)

dans laquelle

$R^1$ et $R^2$ ont la signification indiquée plus haut et

Hal représente du chlore ou du brome, avec des pyridinols de formule (III)

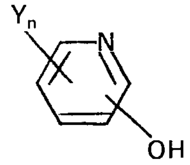

(III)

dans laquelle

Y et n ont la signification indiquée plus haut, en présence d'un agent fixateur d'acide et en présence d'un diluant, ou

b) on fait réagir des halogéno-éther-cétones de formule (IV)

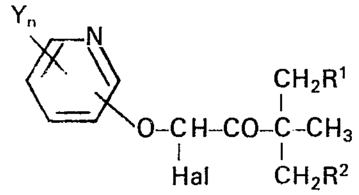

(IV)

dans laquelle

Hal, $R^1$, $R^2$, Y et n ont la signification indiquée plus haut, avec du 1,2,4-triazol en présence d'un agent fixateur d'acide et éventuellement en présence d'un diluant,

c) on réduit éventuellement les céto-dérivés obtenus selon les variantes opératoires (a) et (b) de formule (Ia)

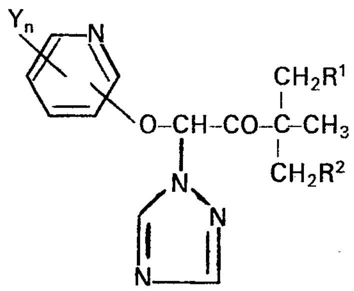

(Ia)

dans laquelle

$R^1$, $R^2$, Y et n ont la signification indiquée plus haut, de la manière usuelle par des méthodes connues, et sur les composés ainsi obtenus de formule (I), on peut ensuite fixer éventuellement un acide ou un sel métallique.

4. Agents fongicides, caractérisés par une teneur en au moins un triazolylalcoyl-pyridyl-éther substitué de formule (I).

5. Utilisation de triazolylalcoyl-pyridyl-éthers substitués de formule (I) pour combattre les champignons phytopathogènes.

## Claims

1. Substituted triazolylalkyl pyridyl ethers of the formula (I)

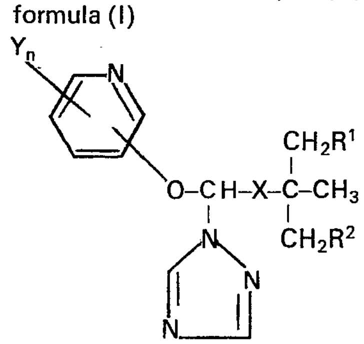

(I)

in which

$R^1$ represents halogen,

$R^2$ represents hydrogen or halogen,

X represents the keto group or a CH(OH) grouping,

Y represents halogen, alkyl or alkoxy with 1 to 4 carbon atoms or cyano and

n represents the number 0, 1, 2, 3 or 4, and physiologically acceptable acid addition salts and metal salt complexes thereof.

2. Substituted triazolylalkyl pyridyl ethers of the formula (I) in Claim 1, wherein

$R^1$ represents fluorine or chlorine,

$R^2$ represents hydrogen or has the same meaning as $R^1$,

X represents the keto group or a CH(OH) grouping,

Y represents halogen, straight-chain or branched alkyl and/or alkoxy with in each case 1 to 4 carbon atoms or cyano and

n represents the number 0, 1, 2, 3 or 4, and physiologically acceptable acid addition salts and metal salt complexes thereof.

3. Process for the preparation of substituted triazolylalkyl pyridyl ethers of the formula (I)

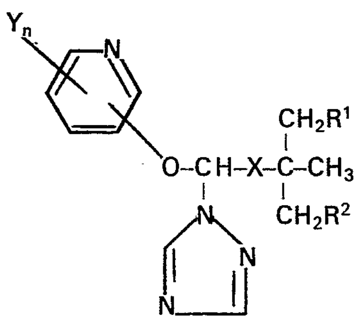

(I)

in which

$R^1$ represents halogen,

$R^2$ represents hydrogen or halogen,

X represents the keto group or a CH(OH) grouping,

Y represents halogen, alkyl, alkoxy or cyano and

n represents the number 0, 1, 2, 3 or 4, and physiologically acceptable acid addition salts and metal salt complexes thereof, characterised in that

a) triazolyl-halogenoketones of the formula (II)

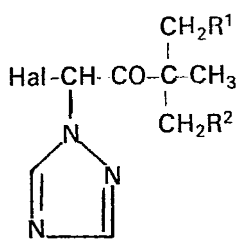

(II)

in which

$R^1$ and $R^2$ have the abovementioned meaning and

Hal represents chlorine or bromine, are reacted with pyridinols of the formula (III)

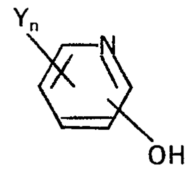

(III)

in which

Y and n have the abovementioned meaning, in the presence of an acid-binding agent and in the presence of a diluent, or

b) halogenoether-ketones of the formula (IV)

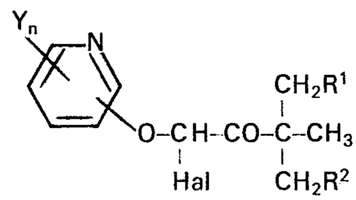

(IV)

in which

Hal, $R^1$, $R^2$, Y and n have the abovementioned meaning, are reacted with 1,2,4-triazole in the presence of an acid-binding agent, and if appropriate in the presence of a diluent; and

c) the keto derivatives obtained by process variants (a) and (b), of the formula (Ia)

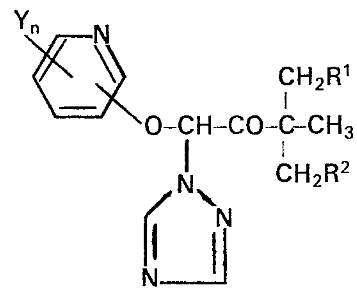

(Ia)

in which

$R^1$, $R^2$, Y and n have the abovementioned meaning, are optionally reduced by known methods in the customary manner, and an acid or a metal salt is then also optionally added onto the compounds of the formula (I) thus obtained.

4. Fungicidal agents, characterised in that they contain at least one substituted triazolylalkyl pyridyl ether of the formula (I).

5. Use of substituted triazolylalkyl pyridyl ethers of the formula (I) for combating phytopathogenic fungi.